(19) Europäisches Patentamt
European Patent Office
Office européen des brevets

(11) Publication number: **0 342 052**
**A2**

(12) # EUROPEAN PATENT APPLICATION

(21) Application number: **89304832.2**

(22) Date of filing: **12.05.89**

(51) Int. Cl.⁴: **C 09 B 69/10**
C 07 K 3/20

(30) Priority: **13.05.88 AR 310844**

(43) Date of publication of application:
**15.11.89 Bulletin 89/46**

(84) Designated Contracting States:
**AT BE CH DE ES FR GB GR IT LI LU NL SE**

(71) Applicant: **Mazza, Jorge Aurelio**
**Santiago del Estero 366**
**Buenos Aires (AR)**

**Outumuro, Pablo**
**Santiago del Estero 366**
**Buenos Aires (AR)**

(72) Inventor: **Mazza, Jorge Aurelio**
**Santiago del Estero 366**
**Buenos Aires (AR)**

**Outumuro, Pablo**
**Santiago del Estero 366**
**Buenos Aires (AR)**

(74) Representative: **Pearce, Anthony Richmond et al**
**MARKS & CLERK Alpha Tower Suffolk Street**
**Queensway Birmingham B1 1TT (GB)**

(54) **Novel monomers for producing coloured polymers; coloured polymers; process for preparing said polymers and for separating substances biologically active trough the use of said polymers.**

(57) A monomer for producing a colored polymer is formed by reacting a monomer with a dye to produce a colored monomer. The colored monomer, which may be acrylate based and include a dye such as reactive blue 2 or reactive red 120, is polymerized or copolymerized with another colored and/or uncolored monomer to produce a colored polymer. The colored polymer is used to purify biologically active substances by, for example, afinity chromatography or electrophoresis.

EP 0 342 052 A2

## Description

### Novel Monomers for Producing Colored polymers; colored polymers; process for preparing solid polymers and for separating substances biologically active through the use of said polymers.

This invention refers to new colored monomers, from which new polymers may be obtained, presenting specific reactivity to some molecules biologically active and not to others, which makes them specifically useful to separate said molecules from the mixtures among them. This separation may be made through any of the known procedures of fractionation, purification, isolation, etc. of these biologically active substances.

Specifically, the invention refers to new monomers and polymers, said polymers being constituted by a series of monomeric units, each of them formed by a dye or a dye derivative combined with an uncolored monomer. This invention also refers to the preparation of these polymers, from the colored monomeric units and the above mentioned dye derivatives, and to the application of these polymers to extract, concentrate, separate, isolate and purify substances, through any of the known processes, containing biologically active substances, specially but not limited to them, such as proteins, peptides, hormones, enzymes, growth and transformation factors, nucleic acids and nucleotides. A special case among these processes is the chromatography and within said discipline, the affinity chromatography.

This invention also refers to the monomeric units which will form part of these new polymers, constituted by dyes and/or dye derivatives combined with traditional monomers and, eventually, with other chemical substances, units which will hereinafter be indicated as colored monomers.

The expression "dye or derivatives thereof" as used, refers to any chemical compound with dyeing power regardless of its value as such when being used as a dye in the traditional processes for coloring and dyeing.

Background of the invention :

The purification of substances containing biologically active molecules, is one of the main problems of modern biotechnology. The synthesis of these substances through the new processes developed during the last years, as for instance, genetic engineering, has the drawback in the fact that the molecules of interest are obtained impurified by other type of molecules which accompany them to a greater or lesser extent. Many of these molecules present, in turn, similar characteristics to those of the molecule to be obtained, which renders difficult the separation thereof.

The full separation of a biologically active substance, specially a protein, from its many impurities, is thus complicated and difficult, and usually presents greater difficulties than the synthesis process which allowed to obtain it.

On the other hand, this requirement of purity is common to all the biologically active substances produced, but it is even greater in the case of substances which will be used as pharmaceuticals for human beings, as for instance insulins or interferons.

Consequently, different techniques, more or less complex, have been developed, aimed at separating the substance of interest from the other substances or impurities accompanying it. The techniques usually used are, among others, the fractional crystallization, the liquid-liquid extraction, the paper, thin layer or column chromatographies; the different electrophoresis processes, and the ionic exchange, high performance liquid (HPLC) and more recently, the affinity and molecular exclusion chromatographies.

Among this group of techniques, there are some which are based on a specific interaction between the substance of interest and an adequate reagent, interaction which does not occur between the other present substances or impurities and the same reagent. The substance of interest is thus fixed to said reagent, while the other substances or impurities do not affix thereto. The product substance of interest/reagent may be then separated from the other substances or impurities not affixed to the reagent. Afterwards, through and adequate treatment, the reagent is separated from the substance of interest, thus obtaining it with a degree of purity much superior than the one one it had in its initial mixture with other substances or impurities.

The products covered by this invention are essentially useful for being applied in all these processes of purification of substances based on this specific reaction, substance of interest/reagent, and will undoubtedly allow and important improvement in these processes.

Among these techniques, usually the chromatographic ones stand out, which, in turn, have different variants, according to the nature of the substance or substances which are to be isolated, purified, etc., and the property or properties of said substance which affect or intervene in the selected technique. For example, the molecular exclusion chromatography is based on the size or geometry of the solutes to be isolated or purified, while the ionic exchange chromatography is based on the electric charge and the acid-base properties of the solute.

In the so-called affinity chromatography (Hay & Dean, Chem. and Ind., 1981, page 726) there is a specific union or bond (non-covalent) of the solute, with other molecule (bond) located between the particles, molecules or matrixes which compose the chromatographic column. For instance, certain enzymes are strongly bonded with its specific coenzymes, in a non-covalent way.

This property is used for isolating an enzyme from (or from other solutes) resorting to the affinity chromatography, covalently linking the specific coenzyme or cofactors (NAD, AMP) of the enzyme to be separated or purified, with an appropriate functional group, located on the surface of the particles which form the support of the chromatographic column ("spacer arms or groups"), and afterwards eluting with a solution

of the free ligand (coenzyme). The same principle grounds the application of other bonds, such as inhibitors, antigens, antibodies, lectines, etc.

As support material usually certain polyssacharides are used, such as agarose and dextrans (for instance, the commercial products called Sepharose or Sephadex, etc.) or polyacryl derivatives (for instance, the commercial products called Bio-Gel, Trysacryl, Ultrogel, etc). Generally, the support material is activated before the operation of fixing the selected ligand, with, for instance, cyanogen bromide. It is the case, in general, of applying the greatest possible relation of activator-support, with the purpose of achieving a high fixation capacity in the chromatographic column.

Lately the resolution power of the affinity chromatography columns has been improved, by applying "spacer arms or groups", aimed at compensating the steric hindrance originated in the size and/or geometry of the biologically active molecules of interest. Said spacer arms (aliphatic groups, such as, for instance, polymethylenic chains of 1 to 17 carbon atoms) are pending groups projecting from the surface of the support particles, at whose free end are bonded the ligand groups (Lowe, C:R:Topics in Enzyme and Fermentation Biotechnology, 5th ed., Wiseman, A: Ellis Horwood, Chichester, 1981, chapter 2).

The resolution power of the affinity chromatography has been unexpectedly improved, due to the discovery of a high affinity between dyes and biologically active molecules, specially proteins, which has led to the fractioning or these biologically active molecules by affinity chromatography, using supports and/or colored matrices, as disclosed for instance in:

Eur. Pat. Sol. EP 65286 (Boehringer Mannheim Gmbh)
Eur. Pat. Sol. EP 183198 (Asahi Chemical Ind. Co. Ltd.)
PCT Int. Sol. WO 84/4309 (Pharmacia AB)
Eur. Pat. Sol. EP 94672 (Toray Industries Inc.)
Eur. Pat. Sol. EP 27262 (Dupont de Nemours E.I. and Co.)
USSR SU 1168564 (All Union Scientific Research Inst. of applied Microbiology)
Ger. (East) DD 204 (Akademie der Wissenschaften der DDr)
Eur. Pat. Sol. EP 64378 (Public Health Laboratory Service Board)
USSR SU 979364 (Pirogov, N.I.: Moscow State Medical Inst.)
Brit GB 1602432 (United Kingdom AtOMIC Energy Authority)
Brit. UK Pat. Sol. GB 2053926
US 4546161 (United Kingdom Secretary of State for Defense)
Eur. Pat. Sol. EP 209284 (Imperial Chemical Industries)

Therefore, the previous art has disclosed the affinity of certain dyes and of certain matrices with dyes affixed thereto, with substances with biological activity, such as proteins, peptides, hormones, enzymes, growth and transformation factors, nucleic acids and nucleotides. This affinity has been applied for the fractioning, purification, etc., of these substances, using columns or matrices in which the dye molecules are affixed on an originally uncolored matrix (formed from agarose, dextrans, polyacrylates or polyacrylamides, etc. and the combinations and variants thereof), either directly or with interposition of "spacer arms or groups"-(Lowe-Supra). These spacer arms allow a greater steric freedom in the proximity of the ligand.

## Summary of the invention

This invention is based on the fact that it is possible to polymerize traditional monomers, previously linked to dyes or derivatives thereof, and eventually also to "spacer arms" or other chemical groups located between the dye and the monomer moiety.

The polymerization of these colored monomers originates products which include in their chemical structure the dyes which were present in the initial monomer.

This constitutes a great difference with all what is known until now, which is based on the link of a dye with a previously formed uncolored polymer.

Colored monomers can also be copolymerized with other acryl- or vinyl- or allyl type monomers to obtain copolymers with special properties. Said polymers can be obtained in combination with crosslinking agents (e.g. bifunctional acrylic derivative) to obtain insoluble reticulated colored polymers.

On the other hand, different colored monomers may be mixed, so that mixed polymers may be obtained, based on different traditional monomers and on different dyes, thus being able to vary at will the properties of the final products to be obtained.

Therefore, the new monomers (called colored monomers), which structure includes a polymerizable function, chemically linked with a dye or a derivative thereof, are a subject-matter of this invention.

Another subject-matter of this invention are the new colored monomers, from dyes or derivatives thereof, which have affinity with the substance of interest to be purified.

Another subject-matter of this invention are the colored polymers which comprise the polymerized colored monomers.

Another object of this invention, is the incorporation to the colored monomer of "spacer arms or groups" or other structures or chemical functions which increase the affinity of the final polymer towards the substance of interest.

A further object of this invention is the polymerization process of these colored monomers, to form colored polymers specially useful for the selected reaction with biologically active substances.

Another object of this invention are the fractioning, purification, isolation, etc. processes of substances with

biological activity, in which the above mentioned colored polymers intervene. These processes could be any of the already known ones, from a direct. physical mixture of the substance to be purified with the colored polymer, to the most sophisticated techniques of affinity chromatography.

Among the substances with biological activity above mentioned, the following are specially relevant: proteins and peptides, hormones, enzymes, growth and transformation factors, nucleic acids and nucleotides.

In this invention the concept of "dye" includes all known dyes, either included or not in the Colour Index, and all the derivatives thereof.

The concept of "monomer" includes any of the substances homo or copolymerization known, such as vinylic monomers (vinyl acetate, divinylbenzene, styrene, etc.), the acrylic monomers (acrylic or methacrylic acid and its alkyl esters, acrylamides, specially N-substituted acrylamides as N-(2-hydroxy-1,1-bis (hydroxymethyl ethyl) -2-propenamide (CAS RN 13880-05-2) and N,N -methylenebis (2-propenamide) (CAS RN 110-26-9), the allylic monomers (mono- or bifunctional ones) and all the other known monomers.

According to the foregoing, it is understood that in the colored monomer the dye or derivative thereof may be directly bound to the uncolored monomer, or through "spacer arms" or through any other chemical groups. These "spacer arms" and other chemical groups may also be present in other positions of the colored monomer molecule, or else be incorporated to the colored monomers during the polymerization thereof.

For the polymerization, one colored monomer may be used, or else more than one, either alone or accompanied by any other kind of products, such as, uncolored traditional monomers, substances able to produce spacer arms, or any other chemical substance in general.

The final product (colored polymer) may, in turn be used either alone or mixed, combined or diluted with other matrices, with chemical substances or with inert or activating materials, on the sole condition that its concentration be not less than 0.1% of the whole of the mixture or combination.

The polymerization of the colored monomers obtained according to this invention, follows the processes established by the conventional technique. The election of the polymerization process depends, obviously, on the selected colored monomer: polymerization in emulsion, in solution (in acqueous or organic phase), by condensation, polymerization at random or in blocks, etc., with the adequate catalysts and initiators for each case. The polymerization process also depends essentially on the properties desired for the matrix to be obtained: porosity, rigidy, etc.

Detailed Description of the Invention

One of the aspects of this invention makes reference to colored monomers formed by acrylic monomers and by derivatives of the reactive blue 2 and/or reactive red 120 dyes. These colored monomers are polymerized either alone, or in the presence of other monomers. The final properties of the colored polymer to be obtained shall be in relation with the corresponding proportions of the reagents, the presence or absence of other monomers, "spacer arms", etc., as well as with the polymerization conditions applied in each case. These properties may still be modified by the treatment with reagents or convenient processes on the obtained polymer.

Reactive blue 2 is a dye having the formula:

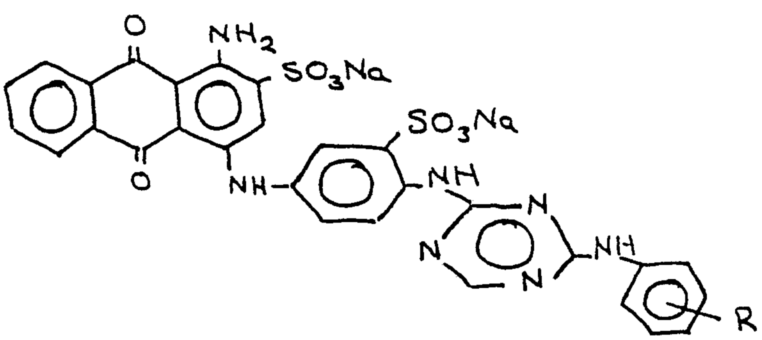

wherein R is an $SO_3Na$ group in the 2-,3- or 4-position, preferably the 2-position; or a derivative thereof.

It should be pointed out in that respect, that the conventional formula accepted for this dye corresponds to a mixture of isomers with the sulfonic group of ring A in position meta and para. Presently it is acknowledged that the dye in fact corresponds to the structure represented in the former formula, i.e. with the sulphonic group in ortho position. However, any of these isomers and the mixtures thereof are usually called "reactive blue 2", name which we adopt in these presents.

Reactive red 120, on the other hand, is an azo dye, which includes in its molecule two triazinic groups, as per the following formula:

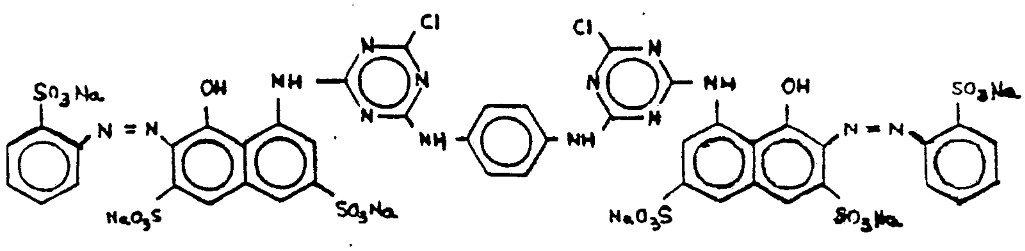

These two dyes are the most mentioned up to this moment by the scientific literature, for the special use of the purification of substances with biological activity through the use of matrices or colored products. In all these cases, the existing literature makes reference to the use of dyes and the derivatives thereof either pure or affixes on an adequate base, but the innovation covered by this patent has not been foreseen, i.e. that same could form part of a colored monomer, from which polymers and derivative substances specially fit for the purification of molecules with biological activity are obtained.

The colored polymers covered by this invention stand out for their capacity for adsorbing and fixing substances (molecules) with biological activity, specially proteins and peptides, hormones, enzymes, and growth and transformation factors, nucleic acids and nucleotides, and for the specificity towards the substance of interest, which is obtained by regulating the above mentioned compounds of the final colored polymer. This adsorption and fixing capacity, as well as this specificity, are superior to those which may be obtained using any of the systems, substances, or mechanisms known up to now.

Thus, for instance, the traditional column of affinity chromatography prepared by dye immobilization on an adequate matrix (Lowe Supra.) has a remarkably inferior capacity than a very similar composition matrix, obtained by polymerization of the corresponding colored monomer.

Example 1

Synthesis of a colored monomer derived from the reactive red 120

The preparation is started from the intermediate compound (I), proceeding according to the following:

Int.(I)

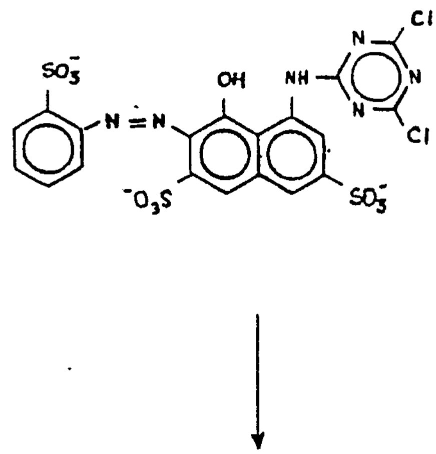

Int.(II)

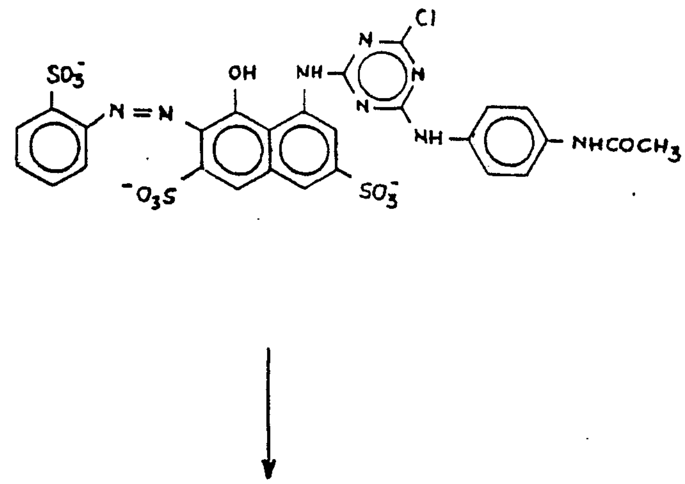

Int.(III)

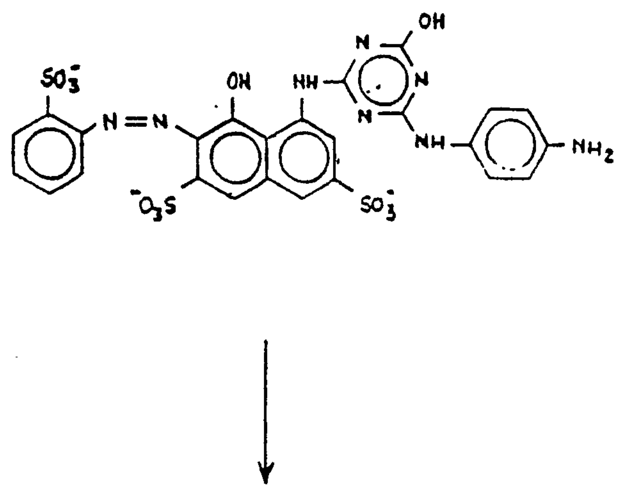

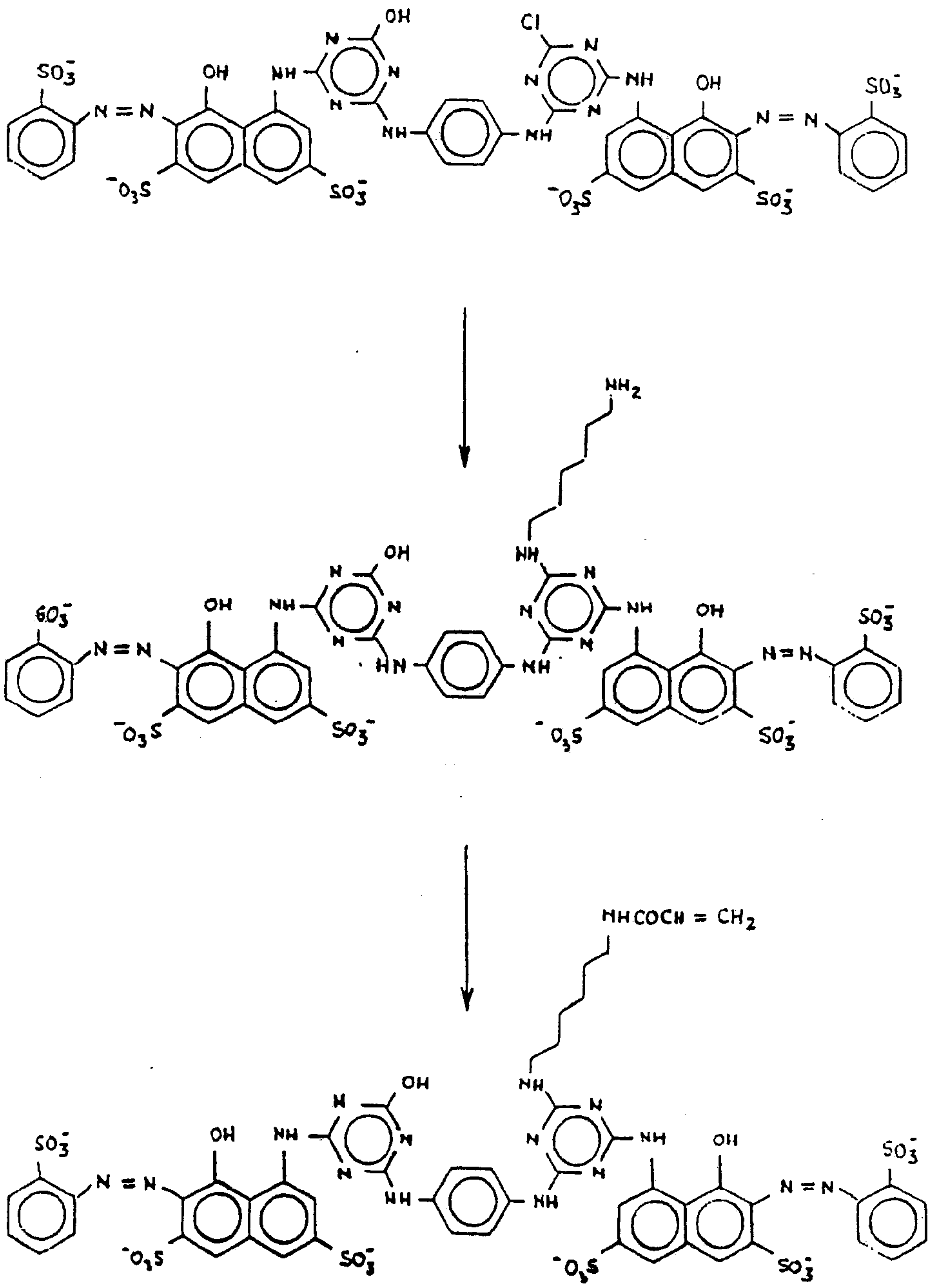

Int. (IV)

Int. (V)

Dye (VI)

A. SYNTHESIS OF THE INTERMEDIATE COMPOUND (II)

To a solution of 0.2 mol of the intermediate compound (I) dissolved in 2500 ml of water, are added 0.22 mol of p-aminoacetanilide in the form of finely divided powder, with mechanical agitation and at room temperature. The pH is kept at 7 by addition of 20% sodium carbonate solution for an hour. Then it is heated to 40°C, maintaining the mechanical agitation, and always regulating the pH at 7 as indicated above.

The achievement of the chemical reaction is checked by paper chromatography for the absence of intermediate compound (I).

B. SYNTHESIS OF THE INTERMEDIATE COMPOUND (III)

To the above solution of the intermediate compound (II) is added a concentrated solution of sodium hydroxide up to pH 13, then the mixture is heated to 95°C maintaining the same temperature for an hour. The end of the reaction is determined by thin layer chromatography on silicagel, verifying the absence of the intermediate compound (II).

The intermediate compound (III) is isolated adjusting the pH to 7, cooling the solution to 5-10 degrees C, and filtering. The filter cake obtained contains the intermediate compound (III) which is directly dried in an oven at

75° C.

C. SYNTHESIS OF THE INTERMEDIATE COMPOUND (IV).

To 0.2 mol of intermediate compound (I) in 2500 ml of water, is added 0.2 mol of the intermediate compound (III) in powder form. The mixture heated to 50-60°c, the pH is kept at 7 with gradual addition of a 20% sodium carbonate solution in water.

Once the reaction ia complete, the pH remains stable. The absence of both free intermediates, compound (I) and (III) is verified by thin layer chromatography. This reaction mixture is used in the following step without isolation.

D. SYNTHESIS OF THE INTERMEDIATE COMPOUND (V).

To the former solution of the intermediate compound (IV) is added 1 mol of hexamethylenediamine and 0.2 mol of pyridine. The mixture is heated to 80° C for an hour, and the absence of the intermediate compound (IV) is verified by thin layer chromatography on silicagel plate.

The intermediate V obtained is isolated after adjusting the pH to 7, cooling to 5-10 degrees C and filtering. The filter cake containing the intermediate compound (V) in a good purity state, is directly dried at 75°C.

E. OBTENTION OF THE ACRYLIC TOSYL ANHYDRIDE.

Solutions in dimethyl formamide of potassium acrylate (or of lithium, sodium or ammonium) and of tosyl chloride are prepared, and are mixed in equimolar proportions of their solutes, at room temperature. A solution of acrylic tosyl anhydride is directly obtained.

The same result could be obtained starting from solutions in dimethyl formamide of potassium tosylate (or of lithium, sodium, or ammonium) and acryloyl chloride, which are mixed in equimolar proportions.

F. SYNTHESIS oF THE COLORED MONOMER (VI)

A solution of 0.2 mol of the intermediate compound (V) is prepared in 2500 ml of water; the pH is adjusted to 9.5 by adding enough quantity of triethylamine; then the solution is cooled to 0-5°C.

To this solution, a solution in dimethyl formamide of acrylic tosyl anhydride obtained according to the description E is added dropwise. The pH is maintained at 9-9.5, by adding, if necessary, some quantity of triethylamine. The addition of acrylic tosyl anhydride solution is ended when the control of reaction through thin layer chromatography shows the absence of intermediate compound (V) in the original solution.

The isolation of the product (IV) is made by adjusting the pH to 5 with acetic acid, adding 5% of potassium chloride and then centrifuging the solution.

The sediment obtained is washed twice by centrifugation in presence of acetone, and is dried in vacuo at room temperature.

The obtained product is one of the colored monomers claimed in this invention, and the polymers of which it may form part constitute other of the claims thereof.

Example 2

Preparation of a coloured matrix

In 10 ml of distilled water and at 20° C, 400 mg of the colored monomer prepared according to the example 1, are dissolved. To that solution 873 mg of N-(2-hydroxy-1,1-bis (hydroxymethyl)ethyl)-2-propenamide (CAS RN 13880-5-2) and 27 mg of N,N -methylenebis(2-propenamide)(CAS RN 110-26-9) are added.

Once obtained the full dissolution, 0.25 ml of an ammomium persulfate at 10% p/p is added, and the polymerization is allowed to progress naturally, working at a temperature between 0 to 50°C. Once finished the polymerization, the obtained substance is washed with distilled water, NaCl M and finally with an hydroalcoholic. mixture. The tridimensional matrix thus obtained is insoluble in water, has a porous form and contains not less than 10 mg or dye per each matrix ml.

Example 3

Reversible adsorption of ovalbumin by a colored matrix

With the colored matrix obtained in the above example, a chromatographic column is formed. A solution of 400 mg of ovalbumin in 10 ml of phosphate buffered saline water is made to circulate through the same. Once ended the adsorption of protein, the column is washed with phosphate buffered saline (or acetate buffer) to eliminate the unbound albumin. The colored matrix is washed with a highly saline solution and the adsorbed ovalbumin is eluted and separated from the matrix. The quantity of reversibly adsorbed ovalbumin is then determined: 10-20 mg of albumin have been reversibly adsorbed per each matrix milliliter.

The preparation of the chromatographic column, the circulation of the liquid to be purified, the separation of the protein adsorbed from the colored matrix and its further titration are made by applying the usual techniques and processes used in this speciality (see, for instance "Affinity chromatography" Indu Parikh and Pedro Cuatrecasas, Chemical and Engineering News, Vol. 63, August 26, 1985, pages 17-32, or else "Affinity Chromatography" by Lowe. C.R. and Dean P.D.G.; Wiley-Interscience, London, 1974; or else, "Reactive dyes in protein and enzyme technology" by Clonis Y.D., Atkinson T., Bruton C.J. and Lowe C.R., the Macmillan Press

Ltd., London, 1987).

Example 4

Synthesis of a colored monomer derived from the "reactive blue 2"

400 mg of reactive blue 2 (A) are dissolved in 10 ml of dimethyl formamide at 40° C. 270 mg of hexamethylendiamine and 40 mg of pyridine are added, and then the temperature is brought to 75°C and kept for 4 to 6 hours.

Then, 0.5-0.8 ml of 10N HCl are added until reaching pH = 2. The solution is agitated for 10 minutes and then 9g of Nacl. dissolved in 40 ml of distilled water, are added. The dye precipitates and is filtered.

The filter cake is resuspended in distilled water at 70°C at pH 2 and it is filtered again to eliminate the hexamethylenediamine excess. The intermediate compound obtained is dried at 70-80°C. This intermediate compound is dissolved in 10 ml of dimethylformamide, in the presence of 3 ml of triethylamine; the temperature is adjusted to 30° C and then 100 mg of acryloyl chloride are added, subsequently stirring for two hours. Once the acrylation of the intermediate compound has been completed, 35 ml of a saturated solution of NaCl is added. The mixture is stirred for 15 minutes. Then, 3.5 ml of 10N HCl are added and the solution is stirred for additional 15 minutes. The obtained product is filtered, and dried at 50° C. In this way, a blue colored monomer is obtained, which corresponds to what has been claimed in this patent.

Note: the same colored monomer may be obtained from the above mentioned intermediate compound, by direct reaction with acrylic tosyl anhydride according to the conditions set forth in point F of the Example 1.

Example 5

Preparation of a blue colored matrix polymer

Starting from the colored monomer prepared according to the former example, a colored matrix polymer is prepared, proceeding as indicated in the Example 2. A polymer containing at least 11 mg of dye per matrix ml is obtained.

Example 6

Reversible adsorption of human serum albumin on a blue colored matrix

With the matrix obtained according to the former example, we proceed as indicated in the Example 3. It is finally found that 10-20 mg of human serum albumin have been fixed per each matrix ml of polymer.

## Claims

1. A monomer having a polymerizable moiety or group, chemically bonded to the dye.

2. A monomer having a polymerizable moiety or group chemically bonded directly to the dye.

3. A monomer according to claim 1, wherein the polymerizable moiety or group is chemically bonded to the dye through a "spacer arm".

4. A monomer according to any preceding claim characterized in that the polymerizable moiety or group is an acrylic derivative, such as acrylamide and its N-substituted derivatives, methyl or ethyl acrylate or methacrylate, acryl nitrile, N-((2-hydroxy-1,1-bis (hydroxy-methyl)ethyl)-2-propenamide, or N,N-methylenebis (2-propenamide).

5. A monomer according to claim 4, characterized in that the polymerizable moiety or group corresponds to a mixture of acrylic derivatives.

6. A monomer according to any preceding claim, characterized in that said dye is a reactive blue 2 with the following chemical constitution:

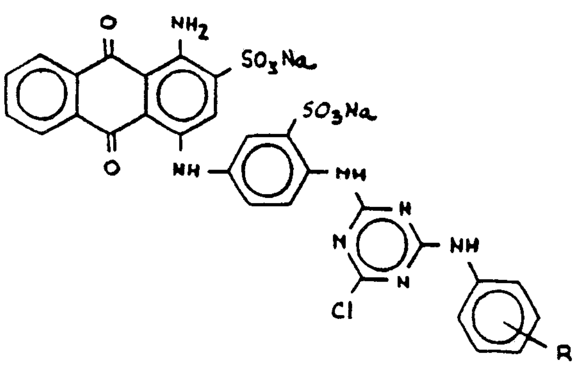

wherein R is equal to a $SO_3Na$ group in position 2, 3 or 4, preferably in position 2; or a derivative thereof.

7. A monomer according to any one of claims 2 to 5, characterized in that said dye is a reactive red 120 of formula

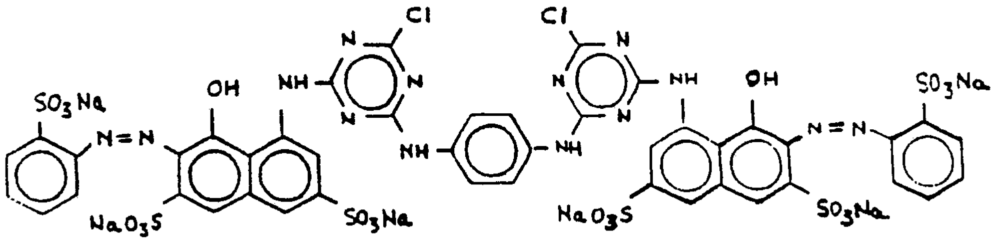

or a derivative thereof.

8. A polymer obtained by polymerization of at least one monomer according to any one of claims 1 to 7, or by copolymerization of said at least one monomer with at least one other monomer.

9. A polymer according to claim 8, obtained by copolymerization of said at least one monomer with at least one different colored monomer.

10. A polymer according to claim 8, obtained by copolymerization of said at least one monomer with at least one uncolored acrylic monomer and, optionally,at least one colored monomer.

11. A polymer according to any one of claims 8 to 10, characterized in that in the molecule of at least one of the colored monomers there is present a spacer arm constituted by a substituted or unsubstituted alkyl chain of 1 to 17 carbon atoms.

12. A process for preparing a polymer comprising the step of polymerising a colored monomer or copolymerizing at least one colored monomer with at least one other monomer.

13. A process according to claim 12, wherein said at least one other monomer is or includes an uncolored monomer.

14. A process for separating a substance with biological activity from impurities, comprising contacting the substance with a colored polymer as claimed in any one of claims 8 to 11, and isolating the substance fixed to said colored polymer.

15. A process as claimed in claim 14, wherein the colored polymer forms part of an affinity adsorbent chromatography or electrophoresis system.

16. The use of a colored monomer as claimed in any one of claims 1 to 7, or a polymer as claimed in any one of claims 8 to 11, in any process or chemical or physicochemical reaction.

17. A method of producing a monomer comprising reacting a compound with a dye to produce a monomer having a polymerizable moiety or a group chemically bonded to a dye.